Europäisches Patentamt

European Patent Office

Office européen des brevets

(11) Publication number: **0 162 319 B1**

## EUROPEAN PATENT SPECIFICATION

(45) Date of publication of patent specification: **19.11.92**

(51) Int. Cl.⁵: **C12N 15/85**, C07K 15/00, C12N 15/10

(21) Application number: **85104976.7**

(22) Date of filing: **24.04.85**

(54) Method of joining heterologous genes.

(30) Priority: **24.04.84 JP 82432/84**

(43) Date of publication of application:
**27.11.85 Bulletin 85/48**

(45) Publication of the grant of the patent:
**19.11.92 Bulletin 92/47**

(84) Designated Contracting States:
**AT BE CH DE FR GB IT LI LU NL SE**

(56) References cited:

**NATURE, vol. 308, 29th March 1984, pages 425-428; S. LEWIS et al.: "Joining of Vk to Jk gene segments in a retroviral vector introduced into lymphoid cells"**

**NATURE, vol. 299, 16th September 1982, pages 265-268, Macmillan Journals Ltd; K. SHIMOTOHNO et al.: "Loss of intervening sequences in genomic mouse alpha-globin DNA inserted in an infectious retrovirus vector"**

**PROCEEDINGS OF THE NATIONAL ACADEMY OF SCIENCES OF THE USA, vol. 79, no. 7,**

**April 1982, pages 2268-2272, Washington, US; J. DOEHMER et al.: "Introduction of rat growth hormone gene into mouse fibroblasts via a retroviral DNA vector: Expression and regulation"**

(73) Proprietor: **Honjo, Tasuku
Higashi-1-jo-agaru, Nishi-iru, Izumidono-cho Yoshida, Sakyo-ku, Kyoto-shi 606(JP)**

(72) Inventor: **Honjo, Tasuku
Higashi-1-jo-agaru, Nishi-iru, Izumidono-cho Yoshida, Sakyo-ku, Kyoto-shi 606(JP)**

(74) Representative: **Füchsle, Klaus, Dipl.-Ing. et al
Hoffmann . Eitle & Partner Patentanwälte Arabellastrasse 4
W-8000 München 81(DE)**

Rank Xerox (UK) Business Services

## Description

The present invention relates to a method of directly joining heterologous genes. More particularly, the invention relates to a method of joining two or more heterologous genes of biologically different origins with their introns eliminated by the splicing technique using a retrovirus vector and a eucaryotic cell.

In higher eucaryotes such as mammals, including man, the RNA transcribed from the operon goes through the process of splicing to become mature mRNA which in turn is translated to a protein. More specifically, the structural gene of an eucaryotic operon consists of alternating exons and introns; the RNA transcribed from each intron is eliminated by splicing to form mature mRNA consisting of only a sequence of RNAs from the exons, and only the protein that is translated from this mature mRNA is produced as the final product. Eucaryotes have the capability of splicing but this is not possessed by procaryotes such as E. coli. Therefore, even if a certain genome gene with introns in a higher animal or plant, for example, a gene coding for a useful physiologically active substance, is inserted into an expression vector for tranformation of a procaryote such as E. coli, the desired physiologically active substance cannot be obtained from the transformed cell.

Therefore, in order to produce in a microorganism such as E. coli a protein that is usually produced by higher animals or plants, the following method is currently used: mature mRNA corresponding to the desired protein is isolated from a cell or tissue capable of producing that protein, and cDNA prepared from the isolated mature mRNA is cloned in a suitable expression vector. Since the cloned gene orinigates from the mature mRNA that has been freed of all intron regions, even a microorganism having no capability of splicing can be used to produce a substance, particularly protein, that is produced by higher animals. As a matter of fact, this method is often used when microorganisms (e.g. E. Coli and yeasts) are employed to produce interferons, growth hormones and many other useful proteins produced by higher organisms.

However, this conventional method requires a considerable amount of purified mRNA. Additionally, in spite of the recent development of a new method of cloning cDNA from mRNA (Okayama, H & Berg, P., Mol. Cell. Biol. 2: 16, 1982), it is sometimes difficult to make cDNA of the desired length.

A method has also been developed that enables the joining in vitro of the exon regions alone in a certain genome by means of chemically synthesized DNA (Adelman, J.P. et al., DNA 2: 183-193, 1983), but if the genome has a large molecular weight and contains many exons, it requires much time and labor to determine and even synthesize the exon regions and their DNA sequence.

Attempts at eliminating introns in a single gene by making use of the splicing capability of a eucaryote have been made by Shimotohno and Temin, who reported the splicing of mouseα -globin gene with the aid of a chick retrovirus (Avian spleen necrosis virus: hereunder abbreviated as SNV) (Shimotohno, K. & Temin, H.M., Nature 299: 265-268, 1982). However, they did not make a detailed study of the DNA sequence of the resulting gene with a view to checking if the introns in the gene had been completely spliced. Additionally, Shimotohno and Temin applied such splicing to the introns only in a single gene and the introns spliced were those naturally occurring in that organism. By the term "single gene" as used hereunder is meant a single gene originating from one organism.

The present inventors therefore made various efforts to develop a method of simultaneously splicing genes originating from two or more organisms by the lifecycles of retroviruses and the splicing capability of eucaryotes. As a result, the inventors have found that their object can be achieved by first inserting genes from two or more different organisms, particularly warm-blooded animals, into a retrovirus-derived DNA vector and by then splicing the DNA with introns in a eucaryotic cell. The other finding of the inventors is that "artificial" introns separating two or more genes within the vector are also spliced to provide the direct joining of these genes. The present invention has been accomplished on the basis of these new findings.

One object of the present invention, therefore, is to provide a method of producing and cloning an intron-free cDNA gene from genomes of higher animals or plants by making use of a retrovirus vector and the splicing capability of a eucaryotic cell without separating and purifying mRNA.

Another object of the present invention is to provide a method for obtaining a single, intron-free gene by splicing two or more genes of different biological origins that are separated by introns within a retrovirus vector.

The intron-free gene obtained by these methods of the present invention can be used for direct production in a eucaryotic cell of a single functional hybrid protein that does not occur in nature and which is composed of the two or more joined proteins encoded by the individual genes subjected to the splicing.

The advantages of joining genes of biologically different origins in accordance with the methods of the present invention are particularly great when they are applied to joining immunoglobulin genes. By using the present invention, a gene encoding a hybrid immunoglobulin protain whose molecular structure originates partly from man and partly from another animal can be obtained. By expressing this gene in a

EP 0 162 319 B1

microorganism, a hybrid immunoglobulin protein can be prepared that has both a mouse-derived variable region (V region) having specificity to a particular antigen and a human-derived constant region (C region) which has species-specific functions and activates the immune system of the human body. This protein can be used as a monoclonal antibody for the purpose of curing or diagnosing diseases in human beings without causing any undesired antigen-antibody reaction.

The demand for developing human monoclonal antibodies for use in the treatment or diagnosis of diseases in humans is increasing today. However, it is practically impossible to establish cells that are capable of consistent production of human monoclonal antibodies having the desired antigen specificity. Even if such cells were established, their malignant nature would, for safety reasons, preclude administering the produced antibodies to humans. On the other hand, mouse monoclonal antibody-producing cells (including hybridomas) can be established with relative ease, but administering such antibodies to humans is also not recommended since their constant region (C region) is immunogenic and does not have the ability to activate the human immune system.

One possible method for solving these problems would be to use a non-naturally occurring hybrid immunoglobulin protein wherein the in region of a mouse immunoglobulin having the desired antigen specificity is joined to the C region of a human immunoglobulin having the ability to activate the human immune system. However, no practical method is available today that is capable of producing such hybrid immunoglobulin protein.

Therefore, still another object of the present invention is to provide a method by which human and mouse genes respectively encoding for the above described C and V regions are exclusively joined in their exon regions. The joined genes can be used for producing the hybrid immunoglobulin protein by genetic engineering techniques.

Genes encoding the C region of human immunoglobulins can be separated from the genome of any human cells and clones of such genes are already available. Genes coding for the V region having the desired antigen specificity can also be separated with relative ease from a hybridoma produced by fusion of malignant cells and the mouse spleen cells which are sensitized by that antigen. The present invention has been accomplished on the basis of this technical background and provides a process for producing a new gene, as well as a process for producing a hybrid protein useful in biological fields, particularly for curing and diagnosing diseases in humans.

Fig. 1 shows the procedure for producing a retrovirus recombinant DNA, $pSNV-V_{T15}+hC_\gamma l$;

Fig. 2 is a restriction map for a mouse $V_{T15}$Xbal DNA fragment (4.75 kb), which also shows a BamHI fragment used as a probe (enclosed in rectangles);

Fig. 3 is a restriction map for a human $hC_\gamma l$ Hind III-HhaI fragment (2.2 kb), which also shows a SacII fragment used as a probe (enclosed in rectangles);

Fig. 4 is a restriction map for the recombinant region of $pSNV-V_{T15}+hC_\gamma l$ that was obtained as recombinant DNA by the procedure shown in Fig. 1;

Fig. 5 illustrates a syringe for preparing minced tissue from chick embryo;

Fig. 6 shows the results of analyzing spliced recombinant DNA by the Southern transfer method, (a) showing the results obtained by using the $V_{T15}$ BamHI DNA fragment as a probe and (b) showing the results obtained by using the $hC_\gamma l$ SacII DNA as a probe;

Fig. 7 shows the DNA sequencing strategy for the splicing of the BamHI fragment of $pSNV-V_{T15}+hC_\gamma l$, as well as for the areas around joined exons (enclosed in rectangles) in the spliced DNA region; and

Fig. 8(a) to (d) show the DNA sequences, before and after splicing, of the areas around linkages of the exons in Fig. 7, (a) referring to the area around the linkage between $V_HDJ_{H1}$ of $V_{T15}$ and $C_{H1}$ of $hC_\gamma l$, (b) the area around the linkage between $C_{H1}$ and H of $hC_\gamma l$, (c) the area around the linkage between H and $C_{H2}$ of $hC_\gamma l$, and (d) the area around the linkage between $C_{H2}$ and $C_{H3}$ of $hC_\gamma l$.

The method of directly heterologous genes according to the present invention comprises the following steps:

-- inserting two or more DNA sequences of different biological origins into a retrovirus-derived DNA vector, wherein

a) each of said DNA sequences contains a gene to be joined and one or more introns; and wherein

b) the DNA segment situated between the two genes in said vector contains the corresponding splice site recognition sequences in accordance with the GT-AG rule;

-- introducing said vector into eucaryotic cells;

-- cultivating said cells;

-- collecting the culture supernatant containing virus particles from the cells;

-- infecting another lot of eucaryotic cells with the virus particles by contacting the cells with said supernatant; and

3

-- selectively recovering DNA wherein the genes in said two or more DNA sequences have been directly joined by elimination of any DNA segments between the genes and any introns due to the splicing in accordance with the GT-AG rule.

The DNA vector used in the present invention is derived from a retrovirus. The retrovirus is an RNA virus consisting of two homologous RNA molecules encapsulated in a coat protein, and has a life cycle that may be represented by:

$$\rightarrow \text{RNA} \;\; \overset{①}{\rightarrow} \;\; \text{DNA} \;\; \overset{②}{\rightarrow} \;\; \text{RNA} \longrightarrow$$

The characteristic feature of this life cycle is that the virus goes through the same mode of transcription as with the eucaryotic host cell at the stage of DNA $\overset{②}{\rightarrow}$ RNA. Therefore, if for some reason an intron exists in the genome DNA of that virus, it is subjected to the splicing action of the host cell at the stage of DNA $\overset{②}{\rightarrow}$ RNA and an intron-free DNA is obtained through the stage of RNA $\overset{①}{\rightarrow}$ DNA. The present invention makes use of this interesting phenomenon and has as its prerequisite the use of a retrovirus-derived DNA vector and eucaryotic cells.

Typical examples of the retroviruses from which the DNA vector used in the present invention can be elicited include chick retroviruses and mouse retroviruses. An advantageous example is the hybrid vector pSNV-TKΔter(R) which is a chick retrovirus-derived DNA vector containing E. coli pBR322 DNA and thymidine kinase gene and which is described in Shimotohno, K & Temin, H.M., Cell, 26, 67-77, 1981 (according to the contribution rules of "Cell", all cell strains and plasmids described in the journal should be freely distributed to a third party who has made a request for research purposes). Another preferred example is a mouse-derived retrovirus such as the Monoly murine leukemia virus (M-MuLV: Herman van der Puptten, et. al., Cell 24: 729-739, 1981).

In principle, the eucaryotic cells used in the present invention may be derived from any eucaryotes. However, if the DNA vector used is derived from a chick retrovirus, chick cells such as chick embryonic fibroblasts are preferably used. If the DNA vector is derived from a mouse retrovirus, cell lines such as mouse SC-I (Hartley, J.W. & Rowe, W. P., Virology 65: 128-134, 1975) may advantageously be used as host cells.

The two or more different genes of biological origin that are expected to be directly by the method of the present invention are any two or more genes or portions of such genes that can be functionally expressed as a result of splicing in eucaryotic cells. The advantages of the method of the present invention are particularly great if the respective genes have different biological origins. Examples of the sets of genes of different biological origins include the gene for the C region of the heavy chain of a human immunoglobulin and the gene for the V region of the heavy chain of a mouse immunoglobulin, as well as the genes for the light chains of the respective immunoglobulins. The method of the present invention will also be applied for the purpose of directly joining various other intron-containing genes at the exon regions.

The genes to be joined may be inserted into the DNA vector by any known techniques using suitable restriction enzymes. Methods are also known for transforming eucaryotic cells by incorporating the vector with the gene inserts in such cells; an illustrative technique is the calcium phosphate method described in Example (3) given later in this specification. In this case, it is preferred that an intact or complete retrovirus DNA is simultaneously incorporated in the cells as a helper virus together with the vector containing the gene inserts. This helper virus produces a viral protein coat for the RNA transcribed from the vector DNA within the cell nucleus. This coat protein enhances the efficiency of the subsequent contagious infection of the eucaryotic cells with the transcribed RNA, thereby increasing the efficiency of the method of the present invention in joining heterologous genes.

The RNA derived from the DNA vector contained in the viral particles obtained in the culture medium of the transfected cells has been freed of any introns by the splicing capability of the cells when said RNA was transcribed from the vector DNA. The eucaryotic cells are infected by contact with the culture medium containing the RNA virus particles, either directly or in a suitable concentrated form. By cultivating the infected cells, a gene wherein the original genes are directly joined without being separated by any intron can be obtained. This desired gene may be selectively recovered by any known method, for example, the method of Hirt et al. described in Example (4) to be given later in this specification.

The methods of the present invention are hereunder described in detail with regard to the case of directly joining two foreign genes of different origins, i.e., the gene for the variable region (V region) of the heavy chain of a mouse immunoglobulin and the gene for the constant region (C region) of the heavy chain

4

of a human immunoglobulin, using a chick retrovirus DNA vector and chick embryonic fibroblasts. However, it should be understood that the scope of the present invention is by no means limited to this particular case.

For instance it is possible to directly join the mouse immunoglobulin gene encoding the constant region (C region) of the immunoglobulin gene with the human immunoglobulin gene coding for the varibale region (V region) of the gene.

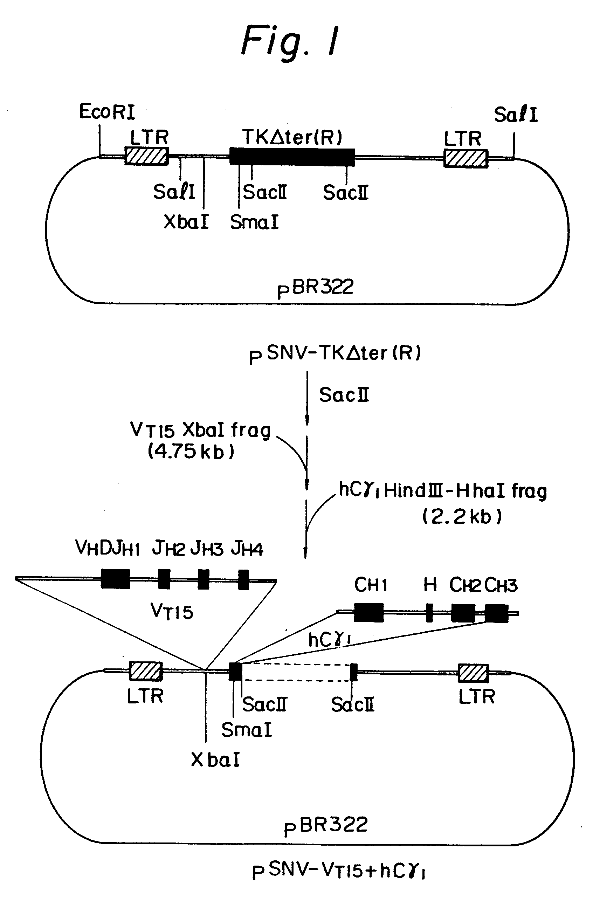

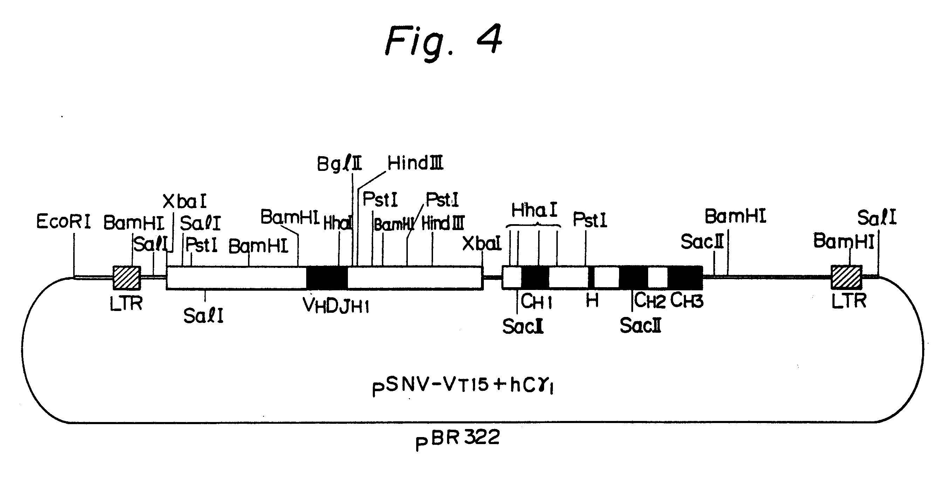

First, as shown in Fig. 1, the retrovirus DNA vector, pSNV-TKΔter(R) DNA, is treated by restriction enzyme SacII so as to have said DNA freed of the SacII DNA fragment of the thymidine kinase gene (TKΔter(R)). Then, 4.75 kb of the $V_{T15}$ XbaI fragment having the V region of the gene in the heavy chain of the mouse immunoglobulin (Nakanishi, K. et al., Proc. Natl. Acad. Sci., USA, 79: 6984-6988, 1982) is inserted into the SacII treated DNA at XbaI site; and thereafter, about 2.2 kb of the hC$\gamma$I HindIII-HhaI fragment having the C region of the heavy chain in the human immunoglobulin gene (Takahashi, N. et al., Cell 29: 671-679, 1982) is inserted at SmaI site. The resulting plasmid pSNV-$V_{T15}$ + hC$\gamma$I has the restriction map depicted in Fig. 4, wherein open rectangle and solid rectangle represent the foreign genes, with solid rectangle particularly referring to the exons.

E. Coli HB101 is then transfected with the plasmid and pSNV-$V_{T15}$ + hC$\gamma$I DNA is separated from cultured cells of the transfected strain. The splicing in chick embryonic fibroblast host cells with this DNA is performed by the following procedures. The purpose of the splicing is to remove from the sSNV-$V_{T15}$ + hC$\gamma$I (see Fig. 4) the DNA sequences (introns) that exist between $V_HDJ_{H1}$ and $C_{H1}$, between $C_{H1}$ and H, between H and $C_{H2}$, and between $C_{H2}$ and $C_{H3}$, thereby obtaining a final DNA as a single gene wherein the original two genes (the V and C regions) are directly together to provide the intron-free sequence of $V_HDJ_{H1} \cdot C_{H1} \cdot H \cdot C_{H2} \cdot C_{H3}$.

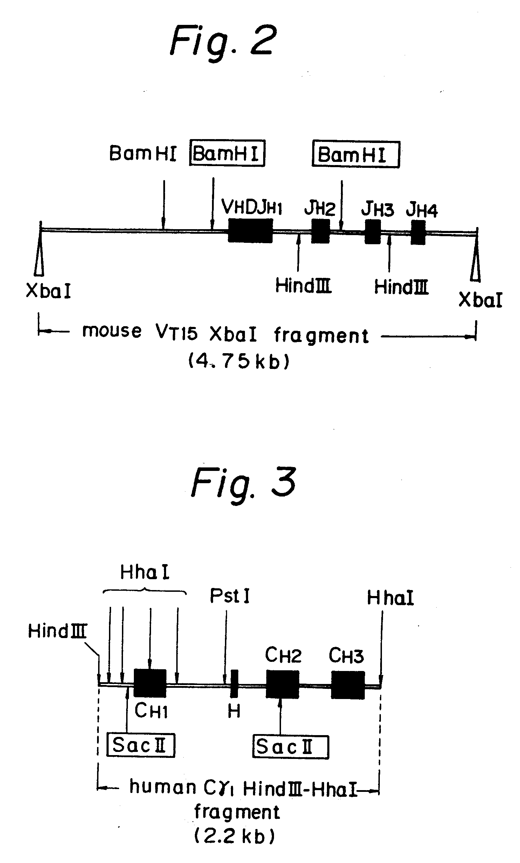

The chick embryonic fibroblasts (hereunder CEF) used in the splicing are prepared from the embryos in commercially available chick eggs of 10 days or so (details of the CEF preparation are described in the Example (2) to be given later in this specification). After 80 - 100% growth, the CEF is transfected by challenge with the previously obtained DNA, pSNV-$V_{T15}$ + hC$\gamma$I, together with a helper virus (e.g. reticuloendotheliosis virus strain A (REV-A)). The occurrence of transfection can be confirmed by measuring the reverse transcriptase activity (R.T. activity) of the virus particles present in the supernatant of the culture of the treated CEF. The transfected CEF cells are cultivated for several days with the culture medium replaced every day. The supernatant of the culture (which contains the RNA virus particles derived from the helper virus DNA and those derived form the spliced PSNV-$V_{T15}$ + hC$\gamma$I DNA) is subsequently used to cause viral infection of CEF. The infected CEF cells are cultured for 2 - 3 days, and the virus DNA produced by reverse transcription from RNA in the CEF is recovered from the culture by the Hirt method (Hirt, B., J. Mol. Biol. 26: 365-369, 1967), so as to obtain the desired intron-free gene. The complete elimination of the introns from the gene may be verified by analyzing the recovered DNA by performing the Southern hybridization (Southern, E.M., J. Mol. Biol. 98: 503-517, 1975) with the $V_{T15}$ X$_{ba}$I fragment and hC$\gamma$I HindIII-HhaI fragment (see Figs. 2 and 3, respectively). According to the analysis made by the present inventors, the intron-free pSNV-$V_{T15}$ + hC$\gamma$I DNA could be recovered only from the CEF that was subjected to viral infection by the supernatant of the culture of CEF that had been transfected by the pSNV-$V_{T15}$ + hC$\gamma$I DNA in the presence of helper virus DNA.

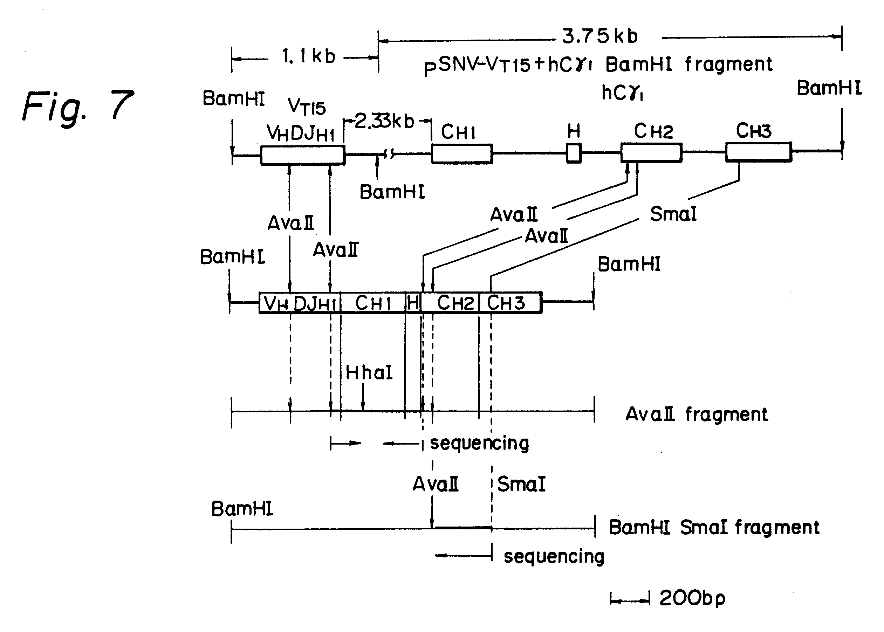

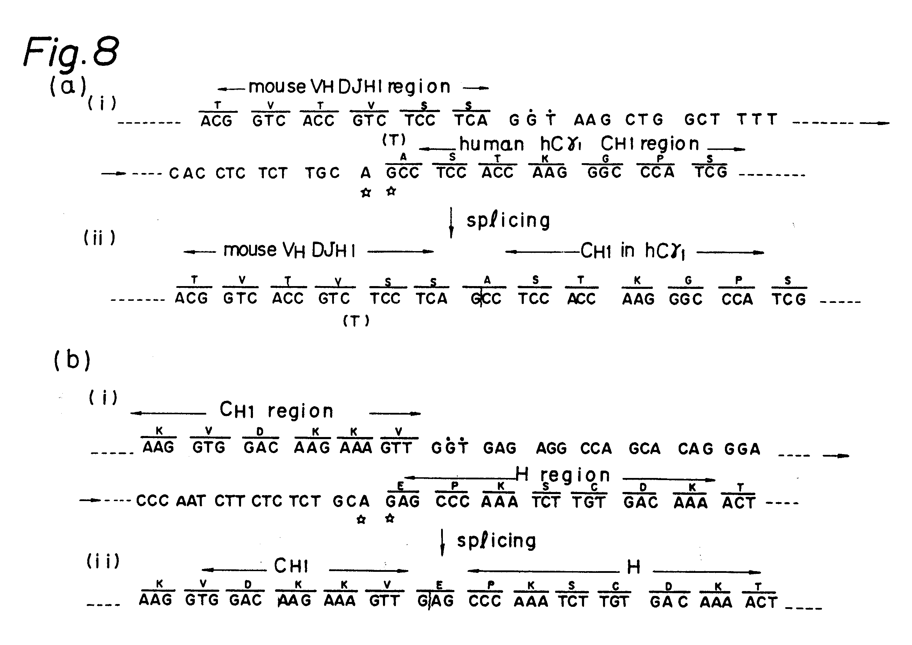

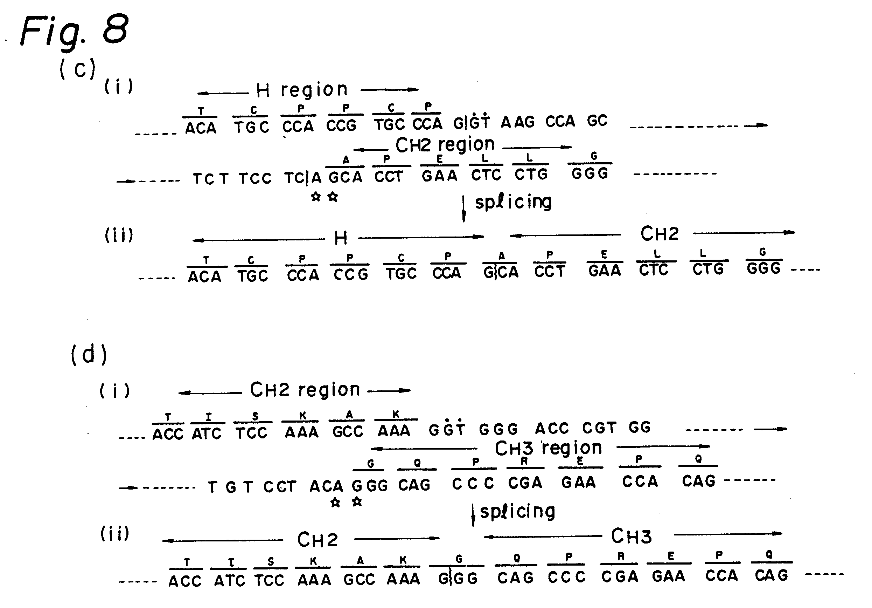

The intron-free DNA was then treated with BamHI to produce about 1.7 kb of the BamHI DNA fragment. This fragment was inserted into a Charon28 vector, packaged in vitro and brought into contact with E. coli LE392 for its infection (E. coli LE392 is a phage host strain commonly used in laboratories). A phage containing about 1.7 kb of the desired DNA fragment was obtained by selecting clones that enter into plaque hybridization with two probes, $V_{T15}$ BamHI and hC$\gamma$I SacII fragments. The base sequence of about 1.7 kb of the BamHI fragment of phage DNA obtained from the selected clones, or that of 1.7 kb of the BamHI fragment of pBR322 DNA obtained by cloning 1.7 kb of the BamHI fragment of said phage DNA, was determined by the Maxam-Gilbert method (Maxam, A. & Gilbert, W., Methods in Enzymology, 65: 499-560, 1980) (see Figs. 7 and 8). As a result, it could be confirmed that the two genes initially inserted into the retrovirus DNA, i.e., the V region of the mouse immunoglobulin gene and the C region of the human immunoglobulin gene (see Fig. 1), had been directly joined to form a single, intron-free gene, by the intended splicing process in accordance with the "GT-AG rule" (Breathnach, R. et al., proc. Natl. Acad. Sci., USA, 75: 4853-4857, 1978).

It was therefore demonstrated that the method of the present invention is very useful in producing a single, intron-free gene from heterologous genes having intervening sequences. Obviously, the obtained gene can express a functional hybrid protein in a procaryote (e.g., E. coli) or a eucaryote (e.g., yeast) by joining said gene to a suitable expression vector.

If the thus obtained hybrid protein is an immunotherapeutic agent, it can be used as an immunodiagnostic or immunotherapeutic agent.

A method of obtaining hybrid immunoglobulin protein comprises the following steps:

-- inserting a hybrid gene into a phage or plasmid vector in such a manner that it can be expressed in a microorganism;

-- transforming a microorganism with said vector; and

- recovering a hybrid immunoglobulin protein from a culture of the transformed microorganism,

said hybrid gene to be inserted being the one that has been obtained by joining immunoglobulin genes of different biological origins in accordance with the method described in any of claims 1 to 11.

The methods of the present invention are hereunder described in greater detail by reference to a specific Example.

Example

(1) Preparation of recombinant DNA, pSNV-$V_{T15}$ + hC$\gamma$l (see Fig. 1)

Five micrograms of DNA, pSNV-TK$\Delta$ter(R) (granted by courtesy of K. Shimotohno; Shimotohno, K & Temin, H.M., Cell 26: 66-67, 1981) were reacted with 10 - 15 units of restriction enzyme SacII (product of Takara Shuzo Co., Ltd.) for 1 hr at 37°C. After electrophoresis on 0.7% agarose, a DNA fragment having a length of 7.2 kb was recovered by the DEAE paper method using DE81 of Whatmann Co. (see Maniatis, T. et al., Molecular Cloning - A Laboratory Manual, p. 473, Cold Spring Harbor Laboratory, 1982), so as to obtain 2.3 $\mu$g of that DNA fragment. A portion (250 ng) of the fragment was reacted with 1 unit of T4 DNA ligase (product of Takara Shuzo Co., Ltd.) for 6 hrs at 15°C in 100 $\mu$l of a conventional ligation solution (50 mM trisHCl (pH 7.4), 10 mM MgCl$_2$, 10 mM DTT, 1 mM ATP: see Dugaiczyk, A. et al., J. Mol. Biol. 96: 171-184, 1975). Using the resulting DNA fragments, E. coli HB101 strain was transformed by the usual method. Colonies having pSNV-TK$\Delta$ter(R) DNA which had been depleted of about 1.6 kb of the SacII-SacII DNA fragment were selected from the transformed cells (selected by using ampicillin resistance as a marker) by subjecting the plasmid DNA of the cultured transformant to agarose gel electrophoresis. The deficient DNA was separated from the selected transformant by the conventional method.

The deficient DNA (5 $\mu$g) was cleaved by treatment with 10 units of restriction enzyme Xba$^{\text{I}}$ (product of BRL Co.) for 1 hr at 37°C, followed by treatment with an alkaline phosphatase (product of Takara Shuzo Co., Ltd.). A portion (270 ng) of the so treated DNA was reacted with 200 ng of a 4.75 kb $V_{T15}$ Xba$^{\text{I}}$ fragment of the variable region (hereunder V region) of a mouse immunoglobulin gene (Nakanishi, K. et al., Proc. Natl. Acad. Sci., USA, 79: 6984-6988, 1982) at 15°C for 1 hr in 10 $\mu$l of a ligation solution (for its composition, see above) in the presence of 0.2 unit of T4 DNA ligase. The reaction mixture was diluted 10-fold with ligation solution. The dilution was subjected to further reaction at 15°C for 6 hrs in the presence of another 0.2 unit of T4 DNA ligase. The resulting DNA was used to transform E. coli HB101 (for the method of ligation, see Dugaiczyk, A. et al., J. Mol. Biol. 96: 171-184, 1975). In order to check to see if the transformed cells (as selected by using ampicillin resistance as a marker) had DNA which involves the intended DNA fragment $V_{T15}$ (see Fig. 1), the cell colonies were transferred onto a nitrocellulose filter and checked for the occurrence of hybridization by using about 1.1 kb of the $V_{T15}$ BamHI fragment containing $V_H D J_{H1}$ (see Fig. 2) as a probe (for the method of colony hybridization, see Maniatis et al., supra, pp. 312-319). Plasmid DNA was separated from the cloned colonies and its restriction map was prepared for the purpose of confirming the correct insertion of the $V_{T15}$DNA fragment. The cloned colonies of the transformed cells were then cultivated in 1,000 ml of L-broth so as to obtain 270 $\mu$g of the desired plasmid DNA.

The DNA was cleaved with restriction enzyme SmaI and at the cleavage site was inserted 2.2 kb of hC$\gamma$l Hind III-HhaI fragment from the constant region (hereunder C region) of a human immunoglobulin gene (Fig. 3; see Takahashi, N. et al., Cell 29: 671-679, 1982) that had been treated with DNA polymerase Klenow fragment (product of BRL Co.) to produce flush ends. The insertion of this flush-ended fragment was accomplished by the method already described hereinbefore. The obtained DNA was used to transform E. coli HB101 cells. The transformed colonies were selected by the colony hybridization method (already described) using hC$\gamma$l SacII fragment DNA (see Fig. 3) as a probe. The correct insertion of the desired fragment was confirmed by preparing a restriction map for the plamid DNA in the transformed cells. Clones of the transformed cells were grown in 3,000 ml of L-broth. By extracting plasmid DNA from the cultured cells and purifying the same, about 800 $\mu$g of the desired recombinant DNA, pSNV-$V_{T15}$ + hC$\gamma$l was obtained (see Fig. 4). The extraction and purification of plasmid DNA were performed by referring to "Idensh Sosa Manual (Gene Manipulation Manual)", pp. 3-10, Kodansha Scientific, 1982. The plasmid DNA obtained in (1) was a recombinant DNA having one type of retrovirus DNA as a vector. This plasmid DNA was used in splicing the introns in $V_{T15}$-hC$\gamma$l DNA by chick embryonic fribroflasts, as shown below.

(2) Preparation and Culture of Chick Embryonic Fibroblasts (CEF)

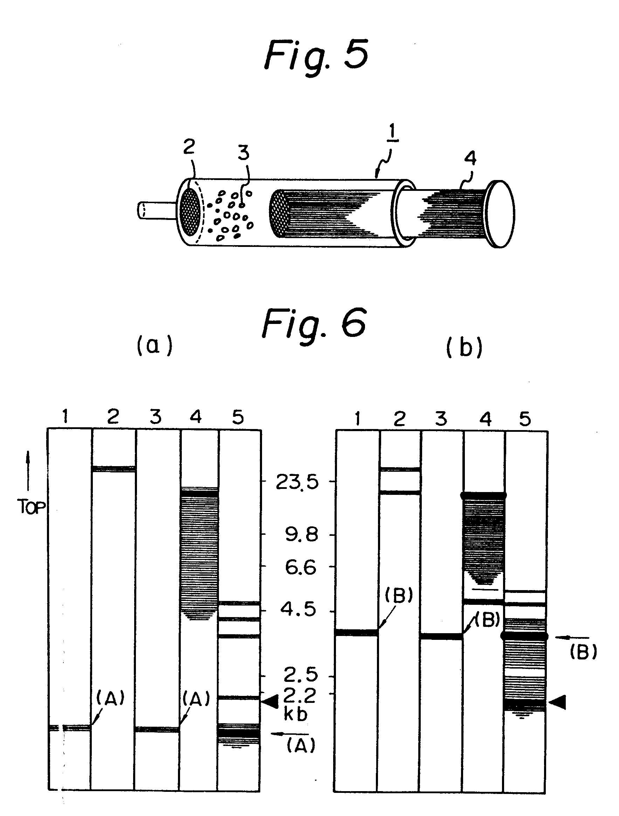

About ten chick embryos were taken from eggs of ten days or so that were purchased from Sato Hatchery, Nakagyoku, Kyoto, Japan. The limbs and head were removed from each embryo with scissors. The humerus, femur, pelvis, vertebra and the intestines were also removed carefully from each embryo. As shown in Fig. 5, the so treated embryos 3 were put into a 50-ml syringe 1 equipped with a screen 2 (mesh openings: ca. 2 mm x 2 mm) at the tip. The piston 4 was pushed forward so as to squeeze the embryos into a 100-ml Erlenmeyer flask through the screen 2. The flask was also added with 10-20 ml of Dulbecco's PBS(-) (product of Nissui Seiyaku Co., Ltd.: a powder composed of NaCl 8 g, KCl 0.2 g, $Na_2HPO_4$ 1.15 g and $KH_2PO_4$ 0.2 g was dissolved in 1,000 ml of thrice distilled water; the solution is hereunder simply referred to as PBS(-)) and the mixture of PBS(-) and the minced embryos was gently stirred with a stirrer for 10 minutes at 37°C. The dispersed cells were collected and mixed with 2-4 volumes of a ice-cooled culture medium (for its composition, see Table 1 below). The remainder of the minced embryos was again mixed with a 0.2% trypsin solution and the mixture was subjected to 2 - 3 cycles of the same procedure. The medium and wet supernatant (cell suspension) were passed through a filter of 4 - 8 sheets of gauze. The filtrate was centrifuged at 1,000 rpm for 2 - 3 minutes and the precipitate (cells) was re-suspended in a fresh culture medium. If sufficient cell precipitate cannot be obtained by a single centrifugation because of the high viscosity of the supernatant, it must be subjected to another cycle of centrifugation after adding 1 - 2 volumes of a medium. By these procedures, ca. $3 \times 10^7$ cells were obtained per single embryo. The cells were mixed with a culture medium to give a final concentration of $8 - 12 \times 10^5$ cells/ml. The suspension was distributed in 15-ml portions among 250-ml plastic flasks (product of Halcon International, Inc.) and cultivated at 37.5°C in 5% $CO_2$.

At the 2nd or 3rd day of cultivation, when the cells formed a monolayer they were washed with PBS(-), followed by treatment with a 0.2% trypsin solution. The so treated cells were suspended in a culture medium and centrifuged at 1,000 rpm for 2 - 3 minutes. The precipitate was suspended in a ice-cooled mixture of 90% medium and 10% DMSO to make 3 ml per 250-ml flask. One milliliter portions of the suspension were immediately distributed among serum tubes (product of Sumitomo Chemical Co., Ltd.) and stored under freezing conditions in liquid nitrogen. For transfection purposes, the frozen CEF cells in a single serum tube were seeded in three 50-ml culture flasks and cultivated to 80-100% growth before use. For infection purposes, the frozen CEF cells in a single serum tube were seeded in one dish (10 cm in dia.) and immediately after the formation of a monolayer, the cells were subcultured to three dishes (dia. 10 cm) and cultivated to 80 - 100% growth before use.

Table 1

| Composition of Culture Medium | |
|---|---|
| Eagle's MEM medium (Nissui ① # 05900), 9.4 g/L, sterilized in autoclave | 400 ml |
| Tryptose phosphate medium (Difco Co., # 0060-01-6), 29.5 g/L, sterilized in autoclave | 100 ml |
| L-glutamine, 200 mM | 5 ml |
| Fetal bovine serum (Gibco Co.) | 25 ml |
| Sodium bicarbonate, 7% V/V | 12.5 ml |
| Stereptomycin, 10 mg/ml; penicillin G, 10,000 U/ml; and amphotericin B, 2.5 $\mu$g/ml | 5 ml |
| A liquid mixture of these components was passed through a membrane filter (0.22 $\mu$m, Millipore Co.) to prepare the desired culture medium. | |

(3) Transfection of CEF with Recombinant DNA, pSNV-$V_{T15}$ + hC$\gamma$I

When they reach 80 - 100% growth in the 50-ml culture flasks, the medium was discarded and CEF cells were washed with PBS(-) once. A cocktail containing 20 $\gamma$/ml of the recombinant DNA, pSNV-$V_{T15}$ + hC$\gamma$I obtained in (1) and 2 $\gamma$/ml of a helper virus DNA, recticuloendotheliosis virus strain A (REV-A) granted by courtesy of Dr. Shimotohno, was prepared by the calcium phosphate method (Graham, F. L. & van der Eb, A.J., Virology 52: 456-467, 1973) and 0.6 ml of this cocktail was added to the recovered CEF monolayer (in 50-ml flask). After leaving the flask for 5 minutes at room temperature, 2.4 ml of a fresh culture medium having the composition shown in Table 1 was added, and incubation was conducted at 37°C in 5% $CO_2$. Four hours later, the medium was replaced by a fresh one and after 18 hours of the transfection, the cells were treated in a medium containing 2.5% (V/V) glycerol for 1 minute at room temperature (Nielsen, D.A. et al., Proc. Natl. Acad. Sci., USA, 80: 5198-5202, 1983), washed with PBS(-) twice, and further cultured in a fresh medium.

After 4 or 5 days of the transfection, the activity of reverse transcriptase (hereunder R.T.) in the culture medium was measured (by the method which will be described later in this specification), and the occurrence of the transfection or the presence of viruses in the medium was confirmed by the increased R.T. activity. If the transfection has occurred, the R.T. activity (evaluated in terms of the count of $^3$H-TTP in the TCA insoluble fractions) increases to a level several times as great as with the control. After confirming the establishment of transfection, the supernatant of the culture medium was daily replaced with a fresh medium, and the recovered supernatant of the culture was stored at -80°C.

The R.T. activity measurement was conducted in accordance with the methods of Hagino, K. et al., (Virology 107: 174-179, 1980) except that a mixture of 20 $\mu$l of the medium in the flask and 10 ul of distilled water was initially added to the reaction solution described in that reference.

(4) Infection of CEF with Recombinant DNA and Recovery of Spliced Recombinant DNA

CEF cells that had achieved 50 - 80% growth on a 10 cm$^{\varnothing}$ dish were infected with the recombinant virus by addition of 3 - 4 ml of the culture supernatant recovered in (3). Two or three days after the infection, virus DNA that was reverse transcribed from RNA in the CEF was recovered by the Hirt method (Hirt, B., J. Mol. Biol., 26: 365-369, 1967) and dissolved in distilled water (20 $\mu$l of distilled water per DNA in one dish of a dia. of 10 cm). A few pg (picogram) of the spliced recombinant virus DNA was recovered from said one dish. A gel electrophoresis on 0.8% agarose gel and the subsequent Southern method (Southern, E.M., J. Mol. Biol. 98: 503-517, 1975) were conducted as follows in order to confirm that the recovered recombinant DNA had been properly spliced. The results are shown in Fig. 6(a) and (b).

First, 20 $\mu$l of the spliced recombinant virus DNA was placed on lane 4 in Fig. 6. Another 20 $\mu$l of the virus DNA was placed on lane 5 after is has been reacted with 10 units of BamHI (product of Takara Shuzo Co., Ltd.) at 37°C for 2 hrs. The two portions of the DNA were subjected to 0.8% agarose gel electrophoresis. On lane 1, 200 pg of the unspliced DNA, pSNV-$V_{T15}$+hC$\gamma$I (see Fig. 4) was charged as a control after cleaving with BamHI. On lane 2, 20 $\mu$l of DNA obtained by transfecting CEF with only pSNV-$V_{T15}$+hC$\gamma$I in the absence of helper virus DNA in step (3) was allowed to run as negative control. On lane 3 was caused to run DNA that was obtained by cleaving the DNA on lane 2 with BamHI. The gel was subsequently subjected to the Southern transfer (see Southern, E.M., Supra) to prepare a nitrocellulose filter; this filter was first hybridized with hC 1 probe (see Fig. 3) as shown in Fig. 6(b) and then exposed to a boiling 10 mM tris-HCl buffer (pH 8.0) for 10 minutes so as to remove the hC$\gamma$I probe. Thereafter, the nitrocellulose filter was hybridized with $V_{T15}$ probe (see Fig. 2), as shown in Fig. 6(a). Fig. 6(a) and (b) show that bands having a length of about 1.7 kb (indicated by ◄) and located at the same position were only observed in lane 5. These bands were not observed in the DNA sample that was obtained by transfection with the recombinant DNA (pSNV-$V_{T15}$+hC$\gamma$I: see Fig. 4) in the absence of helper virus DNA (lane 3 in Fig. 6(a) and (b)). It could therefore be concluded that the BamHI DNA fragment occurring on lane 5 in Fig. 6(a) and (b) had derived not directly from the recombinant DNA, pSNV-$V_{T15}$+hC$\gamma$I used in the transfection, but from the corresponding intron-free DNA that had been formed through the transfection of that recombinant DNA which was firstly converted to virus (RNA) which in its turn was reverse transcribed in newly infected CEF. This conclusion is supported by these two facts: the BamHI DNA fragment concerned hybridizes with the two probes, $V_{T15}$ and hC$\gamma$I; and the position of the band for that fragment agrees with the length of about 1.7 kb that is expected to occur when the introns within hC 1 and that between $V_{T15}$ and hC$\gamma$I are eliminated. Thus, it may safely be concluded that, as expected, DNA of about 1.7 kb has been freed of all introns that exist between $V_{T15}$ and hC$\gamma$I (i.e. the DNA wherein the exons shadowed in Fig. 4 have been brought into direct contact to provide the sequence of $V_H DJ_{H1}\cdot C_{H1}\cdot H\cdot CH_2\cdot CH_3$). This assumption was verified conclusively by determination of the base sequence of the DNA fragment concerned, as will be shown later in (6).

Band (A) in Fig. 6(a) corresponds to the length 1.1 kb of BamHI fragment of $V_{T15}$, whereas band (B) in Fig. 6(b) corresponds to the length 3.75 kb of BamHI fragment of PSNV-$V_{T15}$+hC$\gamma$I.

(5) Cloning of Intron-free Recombinant DNA

The DNA obtained in (4) by cultivating the infected CEF in accordance with the Hirt method was dissolved in 20 ul of distilled water per dish of a diameter of 10 cm. After addition of 10 units of BamHI, the solution was held at 37°C for 2 hrs. The solution was then subjected to a gel electrophoresis on 0.8% agarose gel and a small DNA fragment (ca. 1.7 kb) obtained by cutting the spliced $V_{T15}$-$hC\gamma l$ DNA with BamHI was separated by the DEAE paper method (described in Maniatis, T. et al., Supra p 473).

The separated DNA was cloned as follows using modified Charon28 as a vector.

BamHI-BglIII fragment of mouse immunoglobulin gene $S_\gamma 2a$ cloned in pBR322 (Nikaido, T. et al., J. Biol. Chem. 257: 7322-7329, 1982) was used as an arm linker to modify Charon28 (Chin-Ping Liu et al., Science 209: 1348-1353, 1980). The modified Charon28 was cleaved with BamHI, the BamHI DNA fragment (ca. 1.7 kb) previously obtained was inserted at the cleavage site by the conventional method (as described in (1)). The modified Charon28 DNA (1.7 kb) was subjected to in vitro packaging by the method described in Maniatis, T. et al., supra, pp. 264-268 and then brought into contact with E. coli LE392 for infection. The infected E. coli cells were plated and the resulting plaques were subjected to the plaque hybridization (Benton, W. D. & Davis, R.W., Science, 196: 180-182, 1977) using $V_{T15}$ and $hC\gamma l$ as probes (see Figs. 2 and 3, respectively). By selecting the plaques that hybridized with both probes, clones containing the desired BamHI DNA fragment (ca. 1.7 kb) were obtained. The clones were cultivated and DNA was extracted from a solution of the phage produced by the cultured clones. The DNA was treated with BamHI and subjected to a gel electrophoresis on 0.8% agarose. The desired DNA of a length of about 1.7 kb was separated by the DEAE paper method (already described in (1)). A portion (5 ug) of the separated DNA was cleaved with AvaII and SmaI and the DNA sequences on the resulting fragments were determined by the Maxam-Gilbert method (already described).

(6) Proving the Elimination of Introns by Sequencing (see Figs. 7 and 8)

Fig. 7 shows the sequences of immunoglobulin genes on the BamHI DNA fragment of the recombinant DNA, pSNV-$V_{T15}$+$hC\gamma l$, and on the BamHI DNA fragment (ca. 1.7 kb) of that recombinant DNA after splicing. Fig. 7 also shows the strategy for the DNA sequencing of the gene linkages around the BamHI fragment of the spliced recombinant DNA.

The DNA sequences around the linkages between $V_H DJ_{H1}$ and $C_{H1}$, between $C_{H1}$ and H and between H and $C_{H2}$ on the BamHI fragment (ca. 1.7 kb) were determined by the Maxam-Gilbert method in accordance with the strategy shown in Fig. 7 after cleaving said DNA fragment with AvaII followed by further cleaving of the resulting DNA fragment with HhaI . The DNA sequence around the linkage between $C_{H2}$ and $C_{H3}$ was also determined by the Maxam-Gilbert method in accordance with the same strategy after cleaving said DNA fragment with SmaI followed by further cleaving of the resulting DNA fragment with AvaII. The results of the sequence determination are shown in Fig. 8 (a) to (d), which depicts only the sequences around the respective linkages. Fig. 8(a) refers to the DNA sequence around the linkage between $V_H DJ_{H1}$ of $V_{T15}$ and $C_{H1}$ of $hC\gamma l$; Fig. 8(b) illustrates the DNA sequence around the linkage between $C_{H1}$ and H of $hC\gamma l$; Fig. 8(c) describes the DNA sequence around H and $C_{H2}$ of $hC\gamma l$; and Fig. 8(d) shows the DNA sequence around the linkage between $C_{H2}$ and $C_{H3}$ of $hC\gamma l$.

In Fig. 8(a) to (d), (i) refers to the state before splicing and (ii) the state after splicing. The DNA sequences before splicing that contained introns had already been determined (for $hC\gamma l$, see Ellison, J.W. et al., Nucl. Acid. Res. 10: 4071-4079, 1982, and for the $J_{H1}$ region of mouse $V_H DJ_{H1}$, see Early, P. et al., Cell, 19: 981-992, 1980).

The bars over the DNA sequences in Fig. 8 represent triplet codons of amino acids. The respective letters above the bars mean the following amino acids:

A: alanine, C: cysteine, D: aspartic acid, E: glutamic acid, G: glycine, I: isoleucine, K: lysine, L: leucine, P: proline, Q: glutamine, R: arginine, S: serine, T: threonine, V: valine.

The dots over the DNA sequences in Fig. 8 and the asterisks below represent, respectively, donor and acceptor sequences in the GT-AG rule (Breathnach, R. et al., Proc. Natl. Acad. Sci., USA, 75: 4853-4857, 1978).

Each of the vertical wavy lines inserted in the DNA sequences in Fig. 8 denotes the region where two exons are joined.

The data shown in Fig. 8 reveal that the introns were eliminated at any of the linkage sites of the spliced DNA and the exons were joined directly in accordance with the GT-AG rule. More specifically, the introns on the recombinant DNA, pSNV-$V_{T15}$+$hC\gamma l$, shown in Fig. 4 (or Fig. 7) were correctly spliced as intended, whereupon the two genes of different origins (the V region of the mouse immunoblobulin gene and the C region of the human immunoglobulin gene) were joined together to produce a single, intron-free functional gene.

It will be readily understood by those skilled in the art that the resulting DNA, if inserted into a suitable expression vector, can be expressed as a single functional bound protein not only in eucaryotes but also in procaryotes such as E. coli. The expressed bound protein will be useful as an immunodiagnostic or

EP 0 162 319 B1

immunotherapeutic agent.

**Claims**

1. A method of directly joining heterologous genes comprising the following steps:
   -- inserting two or more DNA sequences of different biological origins into a retrovirus-derived DNA vector, wherein
      a) each of said DNA sequences contains a gene to be joined and one or more introns; and wherein
      b) the DNA segment situated between the two genes in said vector contains the corresponding splice site recognition sequences in accordance with the GT-AG rule;
   -- introducing said vector into eucaryotic cells;
   -- cultivating said cells;
   -- collecting the culture supernatant containing virus particles from the cells;
   -- infecting another lot of eucaryotic cells with the virus particles by contacting the cells with said supernatant; and
   -- selectively recovering DNA wherein the genes in said two or more DNA sequences have been directly joined by elimination of any DNA segments between the genes and any introns due to the splicing in accordance with the GT-AG rule.

2. A method according to Claim 1 wherein the DNA sequences of different biological origins contain genes of warm-blooded animals and/or humans.

3. A method according to Claim 2 wherein the genes of different biological origins are immunoglobulin genes.

4. A method according to Claim 3 wherein one of the genes is a mouse immunoglobulin gene and the other is a human immunoglobulin gene.

5. A method according to Claim 4 wherein the mouse immunoglobulin gene encodes the variable region (V region) of the immunoglobulin gene whereas the human immunoglobulin gene codes for the constant region (C region) of the gene.

6. A method according to Claim 4 wherein the mouse immunoglobulin gene encodes the constant region (C region) of the immunoglobulin gene whereas the human immunoglobulin gene codes for the variable region (V region) of the gene.

7. A method according to Claim 1 wherein the vector into which two or more genes with introns are inserted is a hybrid vector comprising a retrovirus-derived DNA and pBR 322 DNA.

8. A method according to Claim 1 wherein the retrovirus is a chick retrovirus.

9. A method according to Claim 1 wherein the retrovirus is a mouse retrovirus.

10. A method according to Claim 1 wherein the eucaryotic cell is a chick cell.

11. A method according to Claim 1 wherein the eucaryotic cell is a mouse cell.

12. A method of obtaining hybrid immunoglobulin protein comprising the following steps:
   -- inserting a hybrid gene into a phage or plasmid vector in such a manner that it can be expressed in a microorganism;
   -- transforming a microorganism with said vector; and
   -  recovering a hybrid immunoglobulin protein from a culture of the transformed microorganism, said hybrid gene to be inserted being the one that has been obtained by joining immunoglobulin genes of different biological origins in accordance with the method described in anyone of the preceding claims.

13. A method according to Claim 12 wherein the microorganism is E. coli or yeast.

**14.** The use of a hybrid immunoglobulin protein as obtained by the method of claims 12 or 13 to prepare an immunodiagnostic or immunotherapeutic agent.

**Patentansprüche**

**1.** Verfahren zum direkten Verbinden heterologer Gene, welches die nachfolgenden Schritte umfaßt:
-- Einfügen von zwei und mehreren DNA-Sequenzen unterschiedlichen biologischen Ursprungs in einen vom Retrovirus abstammenden DNA-Vektor, wobei
a) jede der DNA-Sequenzen ein zu verbindendes Gen und ein oder mehrere Introns enthält; und wobei
b) das zwischen den beiden Genen in dem Vektor angeordnete DNA-Segment die entsprechenden Spleißstellen-Erkennungssequenzen in Übereinstimmung mit der GT-AG-Regel enthält;
-- Einführen des Vektors in eukaryontische Zellen;
-- Kultivieren der Zellen;
-- Sammeln des Kulturüberstandes, der Virusteilchen aus den Zellen enthält;
-- Infizieren einer anderen Menge eukaryontischer Zellen mit den Virusteilchen durch Inberührungbringen der Zellen mit dem Überstand; und
-- selektives Gewinnen von DNA, wobei die Gene in den zwei oder mehreren DNA-Sequenzen durch Eliminierung jedweder DNA-Segmente zwischen den Genen und jedweder Introns aufgrund des Spleißens in Übereinstimmung mit der GT-AG-Regel direkt verbunden worden sind.

**2.** Verfahren nach Anspruch 1, bei dem die DNA-Sequenzen unterschiedlichen biologischen Ursprungs Gene von warmblütigen Tieren und/oder Menschen enthalten.

**3.** Verfahren nach Anspruch 2, wobei die Gene unterschiedlichen biologischen Ursprungs Immunglobulingene sind.

**4.** Verfahren nach Anspruch 3, wobei eines der Gene ein Mausimmunglobulingen und das andere ein Humanimmunglobulingen ist.

**5.** Verfahren nach Anspruch 4, bei dem das Mausimmunglobulingen für die variable Region (V-Region) des Immunglobulingens kodiert, wohingegen das Humanimmunglobulingen für die konstante Region (C-Region) des Gens kodiert.

**6.** Verfahren nach Anspruch 4, bei dem das Mausimmunglobulingen für die konstante Region (C-Region) des Immunglobulingens kodiert, wohingegen das Humanimmunglobulingen für die variable Region (V-Region) des Gens kodiert.

**7.** Verfahren nach Anspruch 1, bei dem der Vektor, in den zwei oder mehrere Gene mit Introns eingefügt werden, ein Hybridvektor ist, der eine vom Retrovirus abstammende DNA und pBR322 DNA enthält.

**8.** Verfahren nach Anspruch 1, bei dem der Retrovirus ein Kükenretrovirus ist.

**9.** Verfahren nach Anspruch 1, bei dem der Retrovirus ein Mausretrovirus ist.

**10.** Verfahren nach Anspruch 1, bei dem die eukaryontische Zelle eine Kükenzelle ist.

**11.** Verfahren nach Anspruch 1, bei dem die eukaryontische Zelle eine Mauszelle ist.

**12.** Verfahren zum Erhalten von Hybridimmunglobulinprotein, welches die nachfolgenden Schritte umfaßt:
-- Einfügen eines Hybridgens in einen Phagen- oder Plasmidvektor auf derartige Weise, daß es in einem Mikroorganismus exprimiert werden kann;
-- Transformieren eines Mikroorganismus mit dem Vektor; und
-- Gewinnen eines Hybridimmunglobulinproteins aus einer Kultur des tranformierten Mikroorganismus, wobei das einzufügende Hybridgen eines ist, das durch Verbinden von Immunglobulingenen unterschiedlichen biologischen Ursprungs in Übereinstimmung mit dem in einem der vorhergehenden Ansprüche beschriebenen Verfahren erhalten worden ist.

**13.** Verfahren nach Anspruch 12, bei dem der Mikroorganismus E. coli oder Hefe ist.

**14.** Verwendung eines gemaß einem der Verfahren der Ansprüche 12 oder 13 erhaltenen Hybridimmunglobulinproteins für die Herstellung eines immundiagnostischen oder immuntherapeutischen Mittels.

**Revendications**

**1.** Procédé de jonction directe de gènes hétérologues comprenant les stades suivants :
-- insertion de deux séquences d'ADN ou davantage d'origines biologiques différentes dans un vecteur d'ADN provenant d'un rétrovirus, dans laquelle :
    a) chacune de ces séquences d'ADN contient un gène à joindre et un ou plusieurs introns; et dans laquelle
    b) le segment d'ADN situé entre les deux gènes dans ce vecteur contient les séquences de reconnaissance du site d'épissage correspondantes conformément à la règle GT-AG;
-- introduction de ce vecteur dans des cellules eucaryotes;
-- culture de ces cellules;
-- collecte du liquide surnageant de la culture contenant des particules de virus provenant des cellules;
-- infection d'un autre lot de cellules eucaryotes par les particules de virus en mettant en contact les cellules avec ce liquide surnageant; et
-- récupération sélective de l'ADN dans lequel les gènes de ces deux séquences d'ADN ou davantage ont été unies directement par élimination des segments d'ADN éventuellement présents entre les gènes et des introns éventuellement présents en raison de l'épissage effectué conformément à la règle GT-AG.

**2.** Procédé selon la revendication 1, dans lequel les séquences d'ADN d'origines biologiques différentes contiennent des gènes d'animaux à sang chaud et/ou des gènes humains.

**3.** Procédé selon la revendication 2, dans lequel les gènes d'origines biologiques différentes sont des gènes d'immunoglobulines.

**4.** Procédé selon la revendication 3, dans lequel un des gènes est un gène d'immunoglobuline de la souris et l'autre est un gène d'immunoglobuline humaine.

**5.** Procédé selon la revendication 4, dans lequel le gène d'immunoglobuline de souris code pour la région variable (région V) du gène de l'immunoglobuline, tandis que le gène de l'immunoglobuline humaine code pour la région constante (région C) du gène.

**6.** Procédé selon la revendication 4, dans lequel le gène de l'immunoglobuline de la souris code pour la région constante (région C) du gène de l'immunoglobuline, tandis que le gène de l'immunoglobuline humaine code pur la région variable (région V) du gène.

**7.** Procédé selon la revendication 1, dans lequel le vecteur dans lequel deux ou plusieurs gènes portant des introns sont insérés est un vecteur hybride comprenant un ADN dérivant d'un rétrovirus et un ADN pBR 322.

**8.** Procédé selon la revendication 1, dans lequel le rétrovirus est un rétrovirus de poulet.

**9.** Procédé selon la revendication 1, dans lequel le rétrovirus est un rétrovirus de souris.

**10.** Procédé selon la revendication 1, dans lequel la cellule eucaryote est une cellule de poulet.

**11.** Procédé selon la revendication 1, dans lequel la cellule eucaryote est une cellule de souris.

**12.** Procédé de préparation d'une immunoglobuline hybride comprenant les stades suivants :
-- insertion d'un gène hybride dans un phage ou un plasmide comme vecteur de telle manière qu'il puisse être exprimé dans un microorganisme;
-- transformation d'un microorganisme avec ce vecteur; et

- récupération d'une immunoglobuline hybride à partir d'une culture du micoorganisme transformé,

ce gène hybride à insérer étant celui qui a été obtenu en joignant des gènes de l'immunoglobuline d'origines biologiques différentes conformément au procédé décrit dans l'une quelconque des revendications précédentes.

13. Procédé selon la revendication 12, dans lequel le microorganisme est E. coli ou une levure.

14. Utilisation d'une immunoglobuline hybride telle qu'obtenue par le procédé selon la revendication 12 ou 13 pour préparer un agent d'immunodiagnostic ou un agent immunothérapeutique.

# Fig. I

# Fig. 2

mouse V$_{T15}$ XbaI fragment
(4.75 kb)

# Fig. 3

human Cγ₁ HindⅢ-HhaI
fragment
(2.2 kb)

# Fig. 4

Fig. 5

Fig. 6

(a)                    (b)

17

Fig. 7

EP 0 162 319 B1

## Fig.8

**(a)**

(i)

← mouse VHDJHI region →

-------- $\overline{ACG}^{T}$ $\overline{GTC}^{V}$ $\overline{ACC}^{T}$ $\overline{GTC}^{V}$ $\overline{TCC}^{S}$ $\overline{TCA}^{S}$ G Ġ Ṫ AAG CTG GCT TTT -------- →

(T) → human hCγ₁ CH1 region →

→ ---- CAC CTC TCT TGC A $\overline{GCC}^{A}$ $\overline{TCC}^{S}$ $\overline{ACC}^{T}$ $\overline{AAG}^{K}$ $\overline{GGC}^{G}$ $\overline{CCA}^{P}$ $\overline{TCG}^{S}$ --------

☆ ☆

↓ spℓicing

(ii)

← mouse VH DJH I →     ← CH1 in hCγ₁ →

------- $\overline{ACG}^{T}$ $\overline{GTC}^{V}$ $\overline{ACC}^{T}$ $\overline{GTC}^{V}$ $\overline{TCC}^{S}$ $\overline{TCA}^{S}$ $\overline{G{\mid}CC}^{A}$ $\overline{TCC}^{S}$ $\overline{ACC}^{T}$ $\overline{AAG}^{K}$ $\overline{GGC}^{G}$ $\overline{CCA}^{P}$ $\overline{TCG}^{S}$ -----

(T)

**(b)**

(i)

← CH1 region →

----- $\overline{AAG}^{K}$ $\overline{GTG}^{V}$ $\overline{GAC}^{D}$ $\overline{AAG}^{K}$ $\overline{AAA}^{K}$ $\overline{GTT}^{V}$ G Ġ Ṫ GAG AGG CCA GCA CAG GGA ---- →

← H region →

→ ---- CCC AAT CTT CTC TCT GCA $\overline{GAG}^{E}$ $\overline{CCC}^{P}$ $\overline{AAA}^{K}$ $\overline{TCT}^{S}$ $\overline{TGT}^{C}$ $\overline{GAC}^{D}$ $\overline{AAA}^{K}$ $\overline{ACT}^{T}$ ----

☆ ☆

↓ spℓicing

(ii)

← CH1 →     ← H →

---- $\overline{AAG}^{K}$ $\overline{GTG}^{V}$ $\overline{GAC}^{D}$ $\overline{AAG}^{K}$ $\overline{AAA}^{K}$ $\overline{GTT}^{V}$ $\overline{G{\mid}AG}^{E}$ $\overline{CCC}^{P}$ $\overline{AAA}^{K}$ $\overline{TCT}^{S}$ $\overline{TGT}^{C}$ $\overline{GAC}^{D}$ $\overline{AAA}^{K}$ $\overline{ACT}^{T}$ ----

EP 0 162 319 B1

# Fig. 8

(c)

(i)

```
              ←———— H region ————→
         T     C     P     P     C     P
----- ——— ——— ——— ——— ——— ——— ————— ——— ——— ——
     ACA   TGC   CCA   CCG   TGC   CCA  G|GT  AAG  CCA  GC    ------------- →
                             ←——— CH2 region ————→
                              A     P     E     L     L     G
—-----  TCT  TCC  TC|A ——— ——— ——— ——— ——— ———  ----------
                       GCA   CCT   GAA   CTC   CTG   GGG
                       ☆  ☆
                                      ↓ splicing
```

(ii)

```
        ←——————————— H ———————————→   ←————— CH2 —————→
         T     C     P     P     C     P     A     P     E     L     L     G
----- ——— ——— ——— ——— ——— ——— ——— ——— ——— ——— ——— ———
     ACA   TGC   CCA   CCG   TGC   CCA  G|CA   CCT   GAA   CTC   CTG   GGG  ----
```

(d)

(i)

```
        ←——— CH2 region ———→
         T     I     S     K     A     K
---- ——— ——— ——— ——— ——— ———
     ACC   ATC   TCC   AAA   GCC   AAA  GGT  GGG  ACC  CGT  GG    ------- →
                                   ←——————— CH3 region ———————→
                                    G     Q     P     R     E     P     Q
—------- T G T  CCT  ACA  GGG  CAG ——— ——— ——— ——— ——— ——— ——— ------
                                    CCC   CGA   GAA   CCA   CAG
                       ☆  ☆
                                      ↓ splicing
```

(ii)

```
        ←————————— CH2 —————————→   ←————— CH3 —————→
         T     I     S     K     A     K     G     Q     P     R     E     P     Q
----- ——— ——— ——— ——— ——— ——— ——— ——— ——— ——— ——— ——— ———
     ACC   ATC   TCC   AAA   GCC   AAA  G|GG   CAG   CCC   CGA   GAA   CCA   CAG  -----
```

EP 0 162 319 B1